# FASCICULE DE BREVET EUROPEEN

(11) **EP 2 595 596 B1**
(45) Date de publication et mention de la délivrance du brevet: **14.01.2015**
(21) Numéro de dépôt: 11743107.2
(22) Date de dépôt: 12.07.2011
(51) Int. Cl.: A61J 3/07, A61K 9/48

(54) **Dispositif et installation d'assemblage d'au moins deux capsules médicamenteuses par collage**
Vorrichtung und Installation zur Verbindung von mindestens zwei Arzneimittelkapseln durch Haftbonden
Device and installation for joining at least two medicine capsules by adhesive bonding

(30) Priorité: 19.07.2010 FR 1055844
(43) Date de publication de la demande: 29.05.2013
(73) Titulaire: Duo-Ge, 92100 Boulogne-Billancourt (FR)
(72) Inventeur: EL GLAOUI, Mehdi, CH-1211 Geneve (CH); EL GLAOUI, Guillaume, F-75116 Paris (FR); HOFFELT, Jean, F-92250 La Garenne Colombes (FR); FONTAINE, Jean-Pierre, F-73100 Aix Les Bains (FR)
(74) Mandataire: Grand, Guillaume
(86) Numéro de dépôt international: PCT/FR2011/051655
(87) Numéro de publication internationale: WO 2012/017149

(56) Documents cités:
- WO-A1-01/03676
- WO-A2-2009/050189

## Description

La présente invention concerne un dispositif et une installation d'assemblage d'au moins deux capsules médicamenteuses par collage.

Dans tout le présent document, le terme « capsule » s'entend comme une forme galénique orale solide, consistant en un mélange d'un principe actif et d'excipients, adjuvants, véhicules et/ou supports. Par forme galénique orale solide, on entend une forme d'administration orale solide, c'est-à-dire une forme habituelle apte à être administrée telle que par voie orale. Cela inclut notamment les formes de comprimé, dragée, gélule ou globule à paroi souple. Ceci exclut une composition pulvérulente ou liquide qui ne serait pas incluse dans une forme galénique orale solide.

Par WO-A-2009/092819, le déposant de la présente a proposé de réunir plusieurs médicaments se présentant sous différentes formes galéniques solides respectives, en une seule forme galénique solide. Un mode de réalisation envisagé pour cette réunion consiste en un assemblage de ces plusieurs médicaments par collage. Une telle forme galénique, résultant du collage de plusieurs éléments galéniques, présente des intérêts significatifs eu égard à la problématique de l'observation des traitements médicamenteux. Ces intérêts sont exposés en détails dans le document précité WO-A-2009/092819, de sorte que le lecteur peut s'y reporter pour plus de détails.

WO-A-01/03676 propose, quant à lui, de fabriquer une forme galénique solide incluant deux compartiments séparés, contenant des poudres médicamenteuses respectives. Pour ce faire, chaque poudre est déposée dans une alvéole d'une courroie propre, avec interposition d'un film rapporté maintenu dans le fond de l'alvéole sous l'action du vide, puis recouverte d'un autre film rapporté. Par entraînement des deux courroies, les deux doses de poudre ainsi filmées sont rapprochées l'une de l'autre, jusqu'à être appliquées l'une contre l'autre avec une colle interposée entre elles.

Le but de la présente invention est de proposer des moyens d'obtention d'un tel assemblage de plusieurs éléments galéniques, qui soient fiables et économiques, en particulier dans un contexte de production industrielle.

A cet effet, l'invention a pour objet un dispositif d'assemblage d'au moins deux capsules médicamenteuses par collage, tel que défini à la revendication 1.

L'idée à la base de l'invention est d'utiliser une matrice souple pour recevoir, centrer et serrer au moins deux capsules distinctes pré-existantes, entre lesquelles de la colle a été déposée. La souplesse de la matrice suivant l'invention est telle qu'on peut la déformer entre deux configurations extrêmes : dans l'une de ces deux configurations, la matrice ouvre largement aux capsules à assembler l'accès à un logement de réception qu'elle délimite et qui est avantageusement conformé pour imposer un positionnement relatif prédéterminé entre les comprimés, en quelque sorte en les empilant, tandis que, dans son autre configuration extrême, la matrice est déformée de manière qu'une paroi de son logement précité épouse davantage les capsules présentes, jusqu'à produire sur elles, par complémentarité de formes, un serrage relatif, vis-à-vis d'un fond du logement, pour comprimer la colle interposée entre elles et ainsi les lier fermement.

En pratique, la matrice souple selon l'invention se présente sous divers modèles et formes de réalisation, comme exposé en détail plus loin. Dans tous les cas, le fait d'utiliser une telle matrice souple pour manipuler les capsules et accompagner leur collage permet d'atteindre des cadences de production importantes, typiquement de plusieurs milliers d'articles par heure, avec des moyens matériels efficaces, fiables et respectueux des contraintes sanitaires et sécuritaires de l'industrie pharmaceutique. En particulier, l'action de la matrice pour aligner et serrer les capsules est douce dans le sens où elle n'endommage pas ces capsules. De plus, la matrice selon l'invention est facilement lavable, notamment à sec. Par ailleurs, dans les limites de ses capacités de déformation élastique, une même matrice peut être utilisée pour assembler deux, voire davantage de capsules présentant des géométries et/ou des dimensions différentes. Autrement dit, l'utilisation de la matrice selon l'invention est modulable et donc économique.

Des caractéristiques avantageuses du dispositif d'assemblage conforme à l'invention, prises isolément ou selon toutes les combinaisons techniquement possibles, sont spécifiées aux revendications dépendantes 2 à 8.

L'invention a également pour objet une installation d'assemblage d'au moins deux capsules médicamenteuses par collage, telle que définie à la revendication 9.

Une mise en oeuvre avantageuse de cette installation est spécifiée à la revendication 10.

L'invention sera mieux comprise à la lecture de la description qui va suivre, donnée uniquement à titre d'exemple et faite en se référant aux dessins sur lesquels :
- la figure 1 est une section schématique d'un dispositif selon un premier mode de réalisation conforme à l'invention ;
- la figure 2 est une vue analogue à la figure 1, illustrant le dispositif dans une configuration de service différente de celle de la figure 1 ;
- la figure 3 est une vue schématique en élévation d'une installation conforme à l'invention, incluant le dispositif de la figure 1 ;
- la figure 4 est une vue schématique en élévation selon la flèche IV de la figure 3;
- la figure 5 est une vue en perspective éclatée d'un second mode de réalisation d'un dispositif conforme à l'invention ; et
- la figure 6 est une coupe dans le plan V de la figure 5, à l'état assemblé du dispositif.

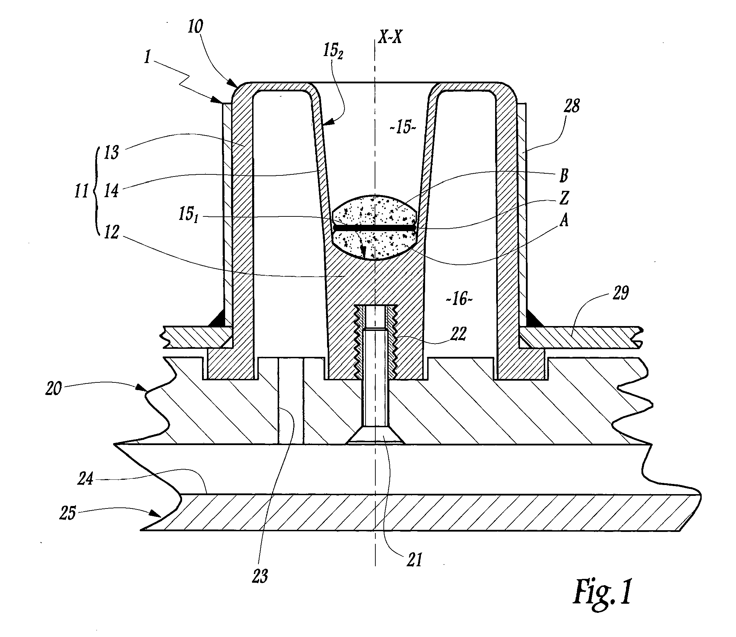

Sur la figure 1 est représenté un dispositif 1 permettant d'assembler l'une à l'autre par collage au moins deux capsules médicamenteuses A et B. Dans l'exemple représenté sur les figures, les capsules A et B présentent des géométries respectives sensiblement identiques, à savoir celles d'un disque présentant une face principale bombée tandis que la face principale opposée est au moins en partie plane pour former entre les capsules A et B une interface de contact plane, avec interposition d'une colle Z sous forme d'une couche. On souligne ici que les formes géométriques respectives des capsules A et B ne sont pas limitatives de la présente invention, dans le sens où d'autres géométries que celles montrées sur les figures sont envisageables pour ces capsules, étant d'ailleurs remarqué que, selon les cas, les capsules A et B peuvent présenter des géométries respectives différentes l'une de l'autre, du moment que les formes retenues puissent établir entre elles une interface de contact où une quantité suffisante de la colle Z est rapportée pour lier fermement l'une à l'autre les capsules A et B.

Comme bien visible sur la figure 1, le dispositif 1 comprend une matrice 10 prévue pour recevoir les capsules A et B. Plus précisément, cette matrice 10 comprend, voire consiste en un corps 11, qui est monobloc dans l'exemple considéré sur les figures et qui est réalisé en un matériau souple, c'est-à-dire un matériau capable de se déformer élastiquement. En pratique, le matériau constituant le corps 11 de la matrice 10 est choisi pour respecter les contraintes sanitaires et sécuritaires de l'industrie pharmaceutique, dans le sens où ce matériau peut être mis en contact avec les capsules médicamenteuses A et B sans risque pour la santé du consommateur ultérieur de ces capsules. En particulier, ce matériau est conforme aux exigences de la « Food and Drug Administration » américaine. A titre d'exemple, ce matériau est du silicone ou à base de silicone.

Le corps 11 de la matrice 10 comporte une embase centrale 12, centrée sur un axe X-X, en étant réalisée ici sous la forme d'un cylindre à base circulaire. Le corps 11 comporte également une paroi périphérique latérale 13, centrée sur l'axe X-X, en étant réalisée ici sous une forme tubulaire à base circulaire. L'embase centrale 12 et la paroi latérale 13 sont reliées l'une à l'autre par une paroi transversale fine 14 appartenant au corps 11, qui s'étend depuis la périphérie d'une des extrémités axiales de l'embase 12 jusqu'à l'une des extrémités axiales de la paroi 13, en courant tout autour de l'axe X-X.

De la sorte, l'embase 12 et la paroi 14 délimitent conjointement un logement 15 dans lequel les capsules A et B peuvent être déposées, comme représenté sur la figure 1. Avantageusement, le fond 15₁ du logement 15, qui est délimité par l'extrémité axiale de l'embase 12, est concave, en étant centré sur l'axe X-X, afin de forcer le centrage sur l'axe X-X des capsules A et B, par effet de glissement contre ce fond 15₁. Avantageusement, ce fond 15₁ épouse au moins partiellement la capsule. De plus, sur son côté tourné vers le logement 15, la paroi 14 présente avantageusement une surface tronconique 15₂, centrée sur l'axe X-X et convergente vers le fond 15₁ du logement 15.

Dans le même temps, l'embase 12, la paroi latérale 13 et la paroi 14 délimitent conjointement, du côté de la paroi 14 opposé au logement 15, une chambre 16 qui court tout autour de l'axe X-X, en entourant le logement 15. Dans l'exemple de réalisation considéré ici, cette chambre 16 présente une géométrie sensiblement annulaire, centrée sur l'axe X-X.

En pratique, la réalisation du logement 15 et de la chambre 16 au sein de la matrice 10 est notamment obtenue par moulage adéquat du corps 11, le moule nécessaire à son moulage étant simple et économique à fabriquer.

De plus, le dispositif 1 comprend un support 20 de fixation de la matrice 10. Dans l'exemple de réalisation considéré ici, ce support 20 est réalisé sous la forme d'une plaque rigide, par exemple métallique ou en matière plastique, dont la face tournée vers la matrice 10 présente des cavités de réception complémentaire des extrémités axiales respectives de l'embase 12 et de la paroi latérale 13, opposées à leur extrémité axiale reliée par la paroi 14. Une vis 21 est rapportée à travers la plaque de support 20, de manière coaxiale à l'embase 12, jusqu'à mettre en prise un insert métallique 22 noyé dans l'embase 12, comme bien visible sur la figure 1.

La plaque de support 20 est également traversée de part en part par un passage libre 23 qui, à l'une de ses extrémités, débouche dans la chambre 16, tandis que son extrémité opposée débouche dans un conduit de distribution pneumatique 24 délimité par une plaque rigide 25, par exemple métallique ou en matière plastique, sur laquelle est montée la plaque de support 20.

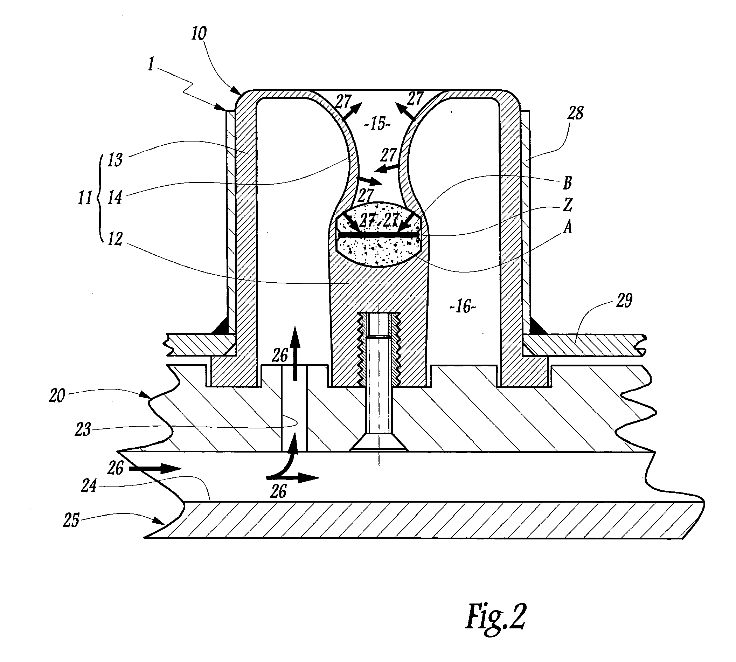

En service, le conduit précité 24 est raccordé à une source pneumatique, non représentée, de préférence une source d'air comprimé. De la sorte, lorsque la source précitée alimente le conduit 24 en air comprimé, ce dernier circule à travers le passage 23, comme indiqué par les flèches 26 sur la figure 2, pour atteindre la chambre 16, provoquant alors l'augmentation de la pression interne de cette chambre. Comme représenté sur la figure 2, sous l'action de cette pression pneumatique, la chambre 16 se gonfle, moyennant la déformation souple de la matrice 10, plus particulièrement de la paroi 14 de son corps 11. Autrement dit, la matrice 10 passe d'une configuration non déformée de son corps 11, représentée sur la figure 1, dans laquelle le logement 15 présente, à l'opposé de son fond 15₁, une ouverture suffisamment grande pour que les capsules A et B accèdent à l'intérieur de ce logement, à une configuration déformée de son corps 11, représentée sur la figure 2, dans laquelle la paroi 14 est élastiquement déformée pour, d'un côté de cette paroi 14, permettre l'expansion de gonflage de la chambre 16 et, du côté opposé, permettre l'étranglement du logement 15, comme indiqué par les flèches 27 sur la figure 2. Eu égard à la présence des capsules A et B dans la zone de fond du logement 15, la paroi 14 se trouve, dans la configuration de la figure 2, déformée en épousant au moins partiellement les capsules de manière à les serrer l'une contre l'autre, en pressant entre elles la colle Z. On comprend que, en raison de la déformation souple et progressive de la paroi 14 lorsque la matrice 10 passe de sa configuration de la figure 1 à sa configuration de la figure 2, les capsules A et B sont progressivement enserrées, du haut vers le bas, entre cette paroi 14 et le fond 15₁ du logement 15, ce qui a pour effet d'aligner les capsules A et B suivant l'axe X-X, puis d'appliquer un pressage relatif entre elles tout en maintenant leur disposition alignée. En pratique, pour commander la déformation progressive de la paroi 14, en accentuant d'abord sa déformation au niveau de l'extrémité évasée du logement 15, puis en propageant cette déformation en direction du fond de logement 15₁, on peut jouer sur une variation d'épaisseur de cette paroi 14.

Le fait de commander le passage de la matrice 10 de sa configuration de la figure 1 à sa configuration de la figure 2 sous l'action d'une pression pneumatique présente l'avantage additionnel que, en cas de crevaison de la chambre de gonflage 16, le corps 11 reste en position non déformée. Par ailleurs, l'utilisation d'air comprimé propre et sec pour gonfler la chambre 16 est une solution économique dans le sens où des sources d'air comprimé sont largement disponibles.

Afin de concentrer sur la paroi 14 l'action de la pression pneumatique régnant dans la chambre gonflée 16, le dispositif 1 comprend avantageusement un raidisseur 28 ceinturant coaxialement la paroi latérale 13, comme représenté sur les figures 1 et 2. En pratique, ce raidisseur 28 est fixé sur une plaque 29 montée sur la plaque du support 20 et participant à la fixation de la matrice 10 à cette plaque de support.

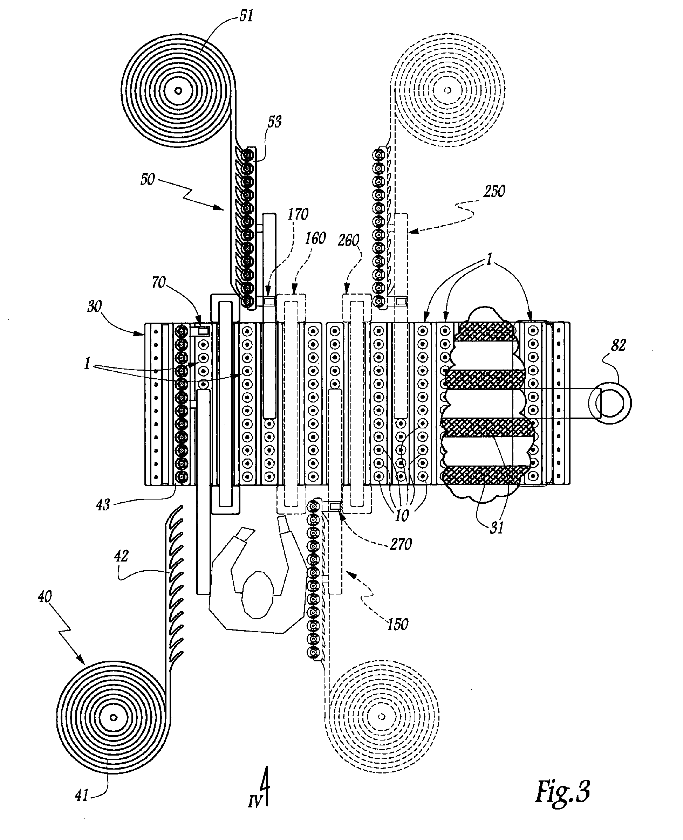

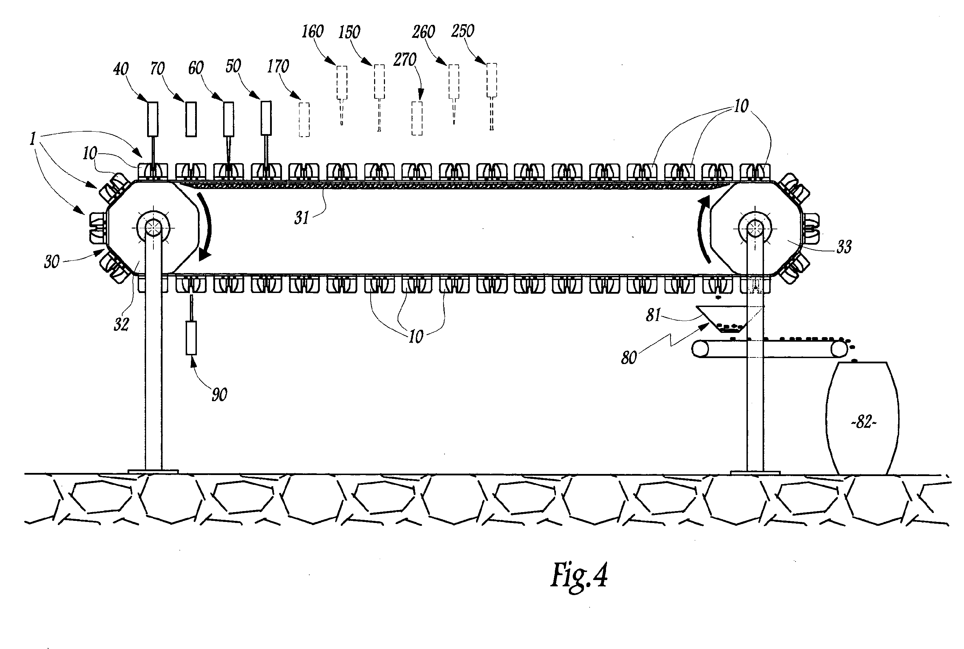

Sur les figures 3 et 4 est représentée une installation comprenant plusieurs dispositifs 1 montés sur un convoyeur 30. Plus précisément, chaque dispositif 1 de cette installation inclut une pluralité de matrices 10 décrites de manière individuelle en regard des figures 1 et 2, ces matrices étant toutes supportées par la même plaque 20, elle-même montée sur la plaque de distribution pneumatique 25 dont le conduit 24 alimente conjointement autant de passages 23 que de matrices 10. Les matrices 10 de chacun de ces dispositif 1 sont ainsi réparties les unes derrières les autres, suivant la direction longitudinale des plaques 20 et 25, comme bien visible sur la figure 3. A titre de variante non représentée, plutôt que de prévoir des matrices individuelles 10 comme décrit juste ci-avant, une seule et même matrice peut être prévue pour s'étendre sur toute la longueur des plaques 20 et 25, cette unique matrice délimitant alors, de manière répartie suivant sa direction longitudinale, plusieurs logements 15 dont chacun est associé à une chambre gonflable 16. Dans tous les cas, lorsqu'on observe le dispositif 1 suivant la direction de l'axe X-X comme sur la figure 3, ce dispositif présente une succession rectiligne de logements 15.

Le convoyeur 30 comprend une ou, comme représenté ici, plusieurs bandes 31 d'entraînement des dispositifs 1, enroulées autour d'un tourteau moteur 32 et, à l'opposé, d'un tambour 33 (figure 4). Par actionnement du tourteau 32, les bandes 31 entraînent les dispositifs 1 qui sont répartis suivant la direction d'entraînement de ces bandes : de la sorte, lorsque l'installation est en service, les matrices 10 de chaque dispositif 1 sont cycliquement tournées vers le haut et tournées vers le bas selon la position d'enroulement des bandes 31 autour du tourteau 32 et du tambour 33, comme bien visible sur la figure 4.

L'installation des figures 3 et 4 comporte également plusieurs postes fonctionnels, répartis autour du convoyeur 30 et agissant sur le dispositif 1 situé à leur niveau respectif, comme détaillé ci-après.

Ainsi, un premier poste 40 est prévu pour déposer dans chaque logement 15 du dispositif 1 situé au niveau de ce poste, alors que la ou les matrices 10 de ce dispositif sont dans leur configuration non déformée de la figure 1, une capsule médicamenteuse A provenant d'une réserve correspondante de ces capsules. Avantageusement, ce poste 40 dépose les capsules A dans le dispositif 1 par action gravitaire. Autrement dit, chaque capsule A tombe à l'intérieur d'un des logements 15, en étant guidé jusqu'au fond 15₁ de ce logement par la surface tronconique 15₂ du logement.

Dans l'exemple de réalisation considéré sur les figures 3 et 4, ce poste 40 comprend un bol vibrant 41 contenant la réserve de capsules A, une rampe 42 de positionnement des capsules A, alimentée en capsules par le bol vibrant 41, ainsi qu'une glissière 43 de préhension des capsules A positionnées par la rampe 42. Préférentiellement, la glissière 43 est conçue pour prendre les capsules A depuis la rampe 42 grâce à un système de ventouse qui, une fois que la glissière 43 est déplacée jusqu'à l'aplomb du dispositif 1 situé au niveau du poste 40, comme représenté sur la figure 3, libère les comprimés A qui tombent alors par gravité dans les logements 15 de la ou des matrices 10 de ce dispositif.

L'installation des figures 3 et 4 comprend également un poste 50 fonctionnellement analogue au poste 40, mais permettant de déposer dans chaque logement 15 de la ou des matrices 10 du dispositif 1 situé au niveau de ce poste 50, non pas une capsule A, mais une capsule B. En pratique, le poste 50 comporte les mêmes éléments que le poste 40, à savoir un bol vibrant 51, une rampe 52 de positionnement des capsules B, et une glissière de préhension 53, étant remarqué que, sur la figure 3, à la différence de la glissière 43 qui est montrée dans sa position à l'aplomb d'un des dispositif 1, la glissière 53 est montrée en cours de coopération avec la rampe de positionnement 52.

Entre les postes 40 et 50 suivant la direction d'entraînement des dispositifs 1 par le convoyeur 30, l'installation des figures 3 et 4 comporte un poste 60 d'application de la colle Z. A titre préférentielle, ce poste 60 inclut un système de libération gravitaire de gouttes de colle, notamment par microdosage, permettant ainsi de faire tomber une goutte de colle Z dans chaque logement 15 de la ou des matrices 10 du dispositif 1 situé au niveau du poste 60. En pratique, eu égard au positionnement du poste 60 par rapport au poste 40, la goutte de colle Z précitée tombe sur la capsule A qui a été préalablement déposée dans chacun des logements 15, lorsque le dispositif 1 était au niveau du poste 40.

A titre d'option avantageuse, l'installation des figures 3 et 4 comporte également un poste 70 qui, suivant la direction d'entraînement des dispositifs 1 par le convoyeur 30, est situé entre les postes 40 et 60 et qui permet de détecter la présence d'une capsule A dans chacun des logements 15 de la ou des matrices 10 du dispositif 1 situé au niveau de ce poste 70. En l'absence d'une ou de plusieurs capsules A, le poste 70 est conçu pour en prévenir un opérateur en vue de procéder à des actions correctives, voire appliquer automatiquement de telles actions : par exemple, le poste 70 peut alors commander le poste aval 60 afin d'inhiber l'application de colle au dispositif 1 concerné.

En service, le convoyeur 30 déplace les dispositifs 1 successivement au niveau des postes 40, 70, 60 et 50, étant précisé que les matrices 10 des dispositifs 1 situés au niveau de ces différents postes sont dans leur configuration non déformée de la figure 1. Puis, après déplacement par le convoyeur 30 du dispositif 1 situé au niveau du poste 50, des moyens de commande, non représentés sur les figures, sont actionnés pour passer la ou les matrices 10 de ce dispositif 1 de leur position non déformée de la figure 1 à leur position déformée de la figure 2 : les capsules A et B présentes dans cette ou ces matrice 10 se trouvent enserrées par le corps 11 de cette ou ces matrices, comme expliqué plus haut en regard de la figure 2. Les moyens de commande précités maintiennent ainsi la ou les matrices 10 du dispositif 1 dans leur configuration déformée pendant que le convoyeur 30 déplace le dispositif 1 jusqu'à placer ce dernier à l'aplomb d'un poste de récupération 80, la ou les matrices 10 de ce dispositif 1 étant alors tournées vers le bas. Le temps nécessaire au dispositif 1 pour être convoyé du poste 50 au poste 80 est ainsi mis à profit pour le séchage de la colle Z interposée entre les capsules A et B présentes dans les logements 15 du dispositif 1. Autrement dit, le secteur du convoyeur 30 s'étendant entre les postes 50 et 80 correspond à un secteur de séchage de la colle Z.

Au niveau du poste de récupération 80, les moyens de commande précités sont désactivés, c'est-à-dire que, par rappel élastique et/ou dégonflage forcé, le corps 11 de la ou chaque matrice 10 du dispositif 1 passe de sa configuration déformée de la figure 2 à sa configuration non déformée de la figure 1 : les capsules A et B collées l'une à l'autre tombent alors dans un pavillon 81 du poste de récupération 80, en sortie duquel les capsules collées sont acheminées jusqu'à un récipient de stockage 82.

A titre d'option avantageuse, l'installation des figures 3 et 4 comporte en outre un poste de lavage 90 (figure 4) permettant de laver, notamment à sec, les logements 15 de la ou des matrices 10 du dispositif 1 situé au niveau de ce poste 90. En pratique, le poste de lavage 90 est situé, suivant la direction d'entraînement des dispositifs 1 par le convoyeur 30, entre le poste de récupération 80 et le poste 40 de dépose de la capsule A.

Ainsi, les dispositifs 1 intégrés à l'installation des figures 3 et 4 permettent d'assembler par collage les capsules A et B de manière fiable et économique, tout en autorisant des cadences de production élevées.

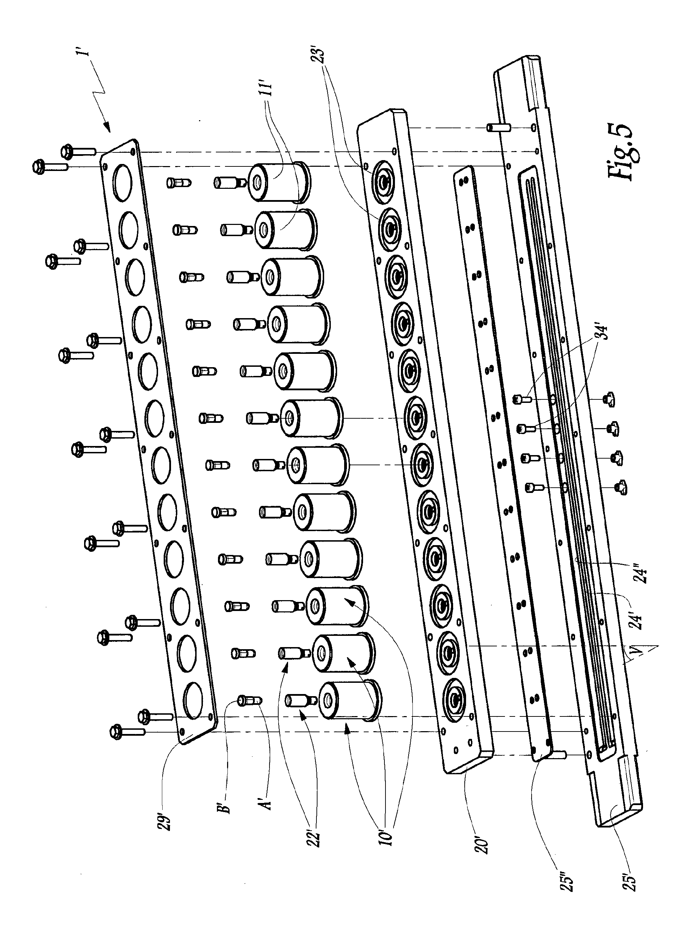

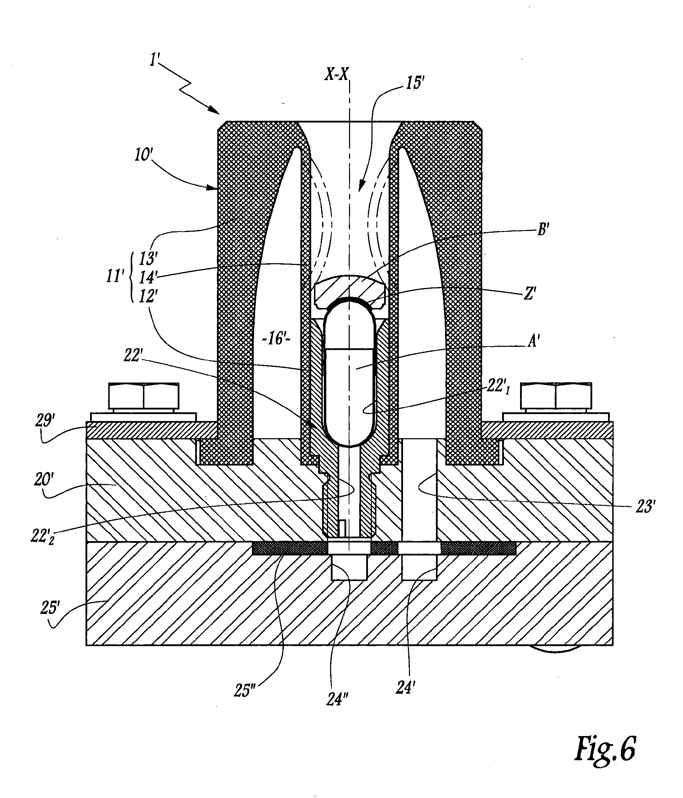

Sur les figures 5 et 6 est représentée une variante de réalisation du dispositif 1, référencée 1'. Ce dispositif 1' peut notamment être intégré à l'installation des figures 3 et 4, en lieu et place de chacun des dispositifs 1. Ci-après, vont être présentées plus en détail les différences entre les dispositifs 1 et 1', étant entendu que les composants du dispositif 1', fonctionnellement similaires aux composants du dispositif 1, portent les mêmes références que ces derniers, suivies de « ' ». Ainsi, le dispositif 1' comporte une pluralité de matrices 10' supportées par une même plaque 20',qui est elle-même montée sur une plaque de distribution pneumatique 25' et à laquelle est assemblée une plaque 29' de fixation des matrices. Chaque matrice 10' consiste en un corps souple 11' incluant une embase centrale 12' et une paroi périphérique latérale 13', ainsi qu'une paroi transversale déformable 14' les reliant l'une à l'autre, toutes les trois centrées sur un axe X-X.

De la même façon que la plaque 25, la plaque 25' est conçue pour être fixée sur le convoyeur 30 ou sur un convoyeur similaire, typiquement à l'aide de vis référencées 34' sur la figure 5.

De manière similaire au dispositif 1, chaque matrice 10' du dispositif 1' délimite une chambre gonflable 16' dans laquelle débouche un passage 23' traversant la plaque de support 20'. Ce passage 23' relie ainsi la chambre 16' à un conduit de distribution pneumatique 24' délimité dans la plaque 25'.

A la différence du dispositif 1, la fixation de chaque matrice 10' à la plaque de support 20' est en partie réalisée par un insert rigide 22' qui est vissé dans la plaque de support 20' depuis la face de cette dernière tournée vers la matrice 10'. Pour ce faire, l'embase 12' de chaque matrice 10' présente, sur toute sa dimension axiale, une forme tubulaire centrée sur l'axe X-X et ouverte à ses deux extrémités. A son extrémité tournée vers la plaque de support 20', l'embase tubulaire 12' débouche sur un taraudage de réception d'une partie filetée de l'insert 22', prévu dans la plaque 20'. Ainsi, moyennant l'introduction, suivant l'axe X-X, de l'insert 22' successivement à l'intérieur de la paroi 14' et de l'embase 12', cet insert est rapporté et vissé à la plaque de support 20', en occupant le volume interne de l'embase 12'. De cette façon, cet insert 22' forme une paroi de fond pour le logement 15' de dépose de capsules A' et B' à coller, délimité intérieurement par la paroi 14'.

Un premier intérêt à cet agencement réside dans le fait que l'insert 22' rigidifie l'embase 12', en maintenant ainsi en place cette dernière.

Un deuxième intérêt est lié au fait que cet insert 22' est avantageusement conformé pour recevoir et caler la capsule A' déposée en premier dans la matrice 10', en épousant au moins partiellement cette capsule A'. Ainsi, dans l'exemple de réalisation considéré sur les figures 5 et 6, chaque insert 22 délimite une cavité allongée 22'₁, centrée sur l'axe X-X et débouchant dans le logement 15', afin de recevoir de manière complémentaire l'essentiel de la capsule A' qui correspond ici à une gélule.

En service, le dispositif 1' s'utilise de la même façon que le dispositif 1. En particulier, lorsque la chambre 16' de chaque matrice 10' est gonflée par de l'air comprimé alimenté par le conduit 24', la paroi 14' passe de sa configuration non déformée, représentée en traits pleins sur la figure 6, à une configuration déformée, représentée en traits mixtes sur la figure 6 et dans laquelle cette paroi aligne puis presse la capsule B', qui s'apparente ici à un comprimé, contre la capsule A', en serrant entre ces capsules de la colle Z'.

Un troisième intérêt lié à l'insert 22' concerne la possibilité de faciliter le dégagement des capsules A' et B' après leur collage. Pour ce faire, comme bien visible sur la figure 6, l'insert 22' délimite intérieurement un canal traversant 22'₂, qui est centré sur l'axe X-X et qui relie sa cavité 22'₁ à un conduit de distribution pneumatique 24" délimité par la plaque 25'. Ce conduit 24" est distinct du conduit 24', en étant étanché vis-à-vis de ce dernier par un joint plat 25" rapporté entre les plaques 20' et 25'. De cette façon, après que les capsules A' et B' aient été collées l'une à l'autre, moyennant l'alimentation en air comprimé du conduit 24', assurant le gonflage de la chambre 16', l'alimentation du conduit 24' est interrompue au profit du conduit 24" : de l'air sous pression circule alors à travers le canal 22'₂ de l'insert 22' de chacune des matrices 10', jusque dans le logement 15', pour éjecter par soufflage les capsules collées A' et B'.

Divers aménagements et variantes aux dispositifs 1 et 1', ainsi qu'à l'installation des figures 3 et 4 sont par ailleurs envisageables. A titre d'exemples :
- pour faciliter le dégagement des capsules collées A et B vis-à-vis de la matrice 10, un canal de soufflage des capsules, fonctionnellement similaire au canal 22'₂ décrit en regard de la figure 6, peut être prévu à travers l'embase 12 ; à l'une de ses extrémités, ce canal de soufflage débouche directement dans le fond 15₁ du logement 15, tandis que son extrémité opposée est raccordée, via les plaques 20 et 25 aménagées en conséquence, à un circuit d'air comprimé distinct de celui alimentant la chambre 16 ;
- plutôt que d'être commandé par une pression, le passage de la matrice 10 ou 10' entre ses configurations non déformée et déformée peut être obtenu par une dépression, c'est-à-dire par aspiration d'air ;
- en jouant sur l'épaisseur de la paroi latérale 13, le raidisseur 28 peut être supprimé, comme c'est le cas pour le dispositif 1' ;
- le poste de récupération 80 peut avantageusement intégrer des moyens de triage des capsules collées qu'il traite, de façon à séparer les produits présumés bons et les capsules considérées comme mal collées ou non conformes ;
- la forme de la matrice 10 ou 10' n'est pas limitée à celle montrée sur les figures ; de même d'autres formes de réalisation que les composants 20, 21, 22, 25 et 29 ou 20', 22', 25' et 29' sont envisageables pour le montage et la commande pneumatique de cette matrice ;
- le nombre de capsules que le dispositif 1 ou 1' peut assembler l'une à l'autre par collage n'est pas limité à deux ; au contraire, trois ou quatre voire davantage de capsules peuvent être collées les unes aux autres grâce au dispositif 1, du moment que chaque logement 15 ou 15' de la ou des matrices 10 ou 10' soit dimensionné en conséquence ; dans ce cas, l'installation des figures 3 et 4 est complétée par autant de postes que besoin, comme indiqué par exemple en traits pointillés sur les figures 3 et 4 : suivant la direction de déplacement des dispositifs 1 par le convoyeur 30, on a ainsi prévu entre les postes 50 et 80, d'une part, la succession d'un poste de détection 170, d'un poste d'application de colle 160 et d'un poste 150 de dépose d'une troisième capsule, qui sont fonctionnellement similaires aux postes 70, 60 et 50, puis, d'autre part, la succession d'un poste de détection 270, d'un poste d'application de colle 260 et d'un poste 250 de dépose d'une quatrième capsule, qui sont eux aussi fonctionnellement similaires aux postes 70, 60 et 50 ; et/ou
- la disposition autour des dispositifs des postes 40, 50, 60 et 70, ainsi que, le cas échéant, des postes 150, 160, 170, 250, 260 et 270 n'est pas limitée à celle montrée sur la figure 3 ; par exemple, pour des raisons d'encombrement, tous ces postes peuvent être agencés d'un seul et même côté du convoyeur 30.

## Revendications

1. Dispositif (1 ; 1') d'assemblage d'au moins deux capsules médicamenteuses (A, B ; A', B') par collage,
**caractérisé en ce qu'**il comprend au moins une matrice (10 ; 10') qui délimite, au moins partiellement, à la fois un logement (15 ; 15') de dépose des capsules (A, B ; A', B') et une chambre (16 ; 16'), ce logement et cette chambre étant séparés l'un de l'autre par une paroi déformable (14 ; 14') de la matrice, laquelle matrice est adaptée pour passer de manière élastique, par action pneumatique de sa chambre (16; 16'), d'une première configuration, dans laquelle son logement (15, 15') reçoit les capsules avec une colle (Z ; Z') interposée entre elles, à une seconde configuration, dans laquelle sa paroi déformable (14; 14') enveloppe, par déformation souple, au moins partiellement les capsules de manière à enserrer au moins partiellement ces capsules entre la paroi déformable et un fond du logement pour les aligner et les presser l'une contre l'autre.

2. Dispositif suivant la revendication 1, **caractérisé en ce que** la matrice (10 ; 10') est adaptée pour passer de sa première à sa seconde configuration sous l'action d'une pression pneumatique qui gonfle la chambre (16 ; 16').

3. Dispositif suivant la revendication 2, **caractérisé en ce que** le logement (15) présente un fond concave (15₁) de centrage des capsules (A, B), qui est délimité par la matrice (10), et **en ce que**, lorsque la matrice est dans sa première configuration, la paroi déformable (14) présente, du côté du logement, une surface (15₂) sensiblement tronconique convergente vers le fond du logement.

4. Dispositif suivant l'une des revendications 2 ou 3, **caractérisé en ce qu'**il comprend un insert rigide (22') de réception et de calage d'une des capsules (A'), qui est agencé dans une embase (12') de la matrice (10'), depuis laquelle s'étend la paroi déformable (14), et qui forme un fond pour le logement (15').

5. Dispositif suivant l'une quelconque des revendications 2 à 4, **caractérisé en ce que** la paroi déformable (14; 14') présente une capacité de déformation souple plus importante que le reste de la matrice (10 ; 10'), qui, à cet effet, est par exemple ceinturé par un raidisseur (28) et/ou présente une plus grande épaisseur et/ou est maintenu en place par l'insert (22').

6. Dispositif suivant l'une quelconque des revendications 2 à 5, **caractérisé en ce qu'**il comprend à la fois un support (20 ; 20') de fixation de la matrice (10 ; 10'), qui délimite un passage (23 ; 23') de gonflage de la chambre (16 ; 16'), et une plaque de distribution pneumatique (25 ; 25'), sur laquelle est monté le support de fixation (20 ; 20') et qui alimente conjointement plusieurs passages de gonflage (23 ; 23') débouchant dans des chambres respectives (16 ; 16') délimitées soit par autant de matrices individuelles (10 ; 10'), soit par une matrice commune.

7. Dispositif suivant la revendication 6, **caractérisé en ce que** la plaque de distribution pneumatique (25') alimente également au moins un canal (22'₂) de soufflage des capsules collées (A', B'), qui débouche dans chaque logement (15').

8. Dispositif suivant la revendication 1, **caractérisé en ce que** la matrice est adaptée pour passer de sa première configuration à sa seconde configuration sous l'action d'une dépression pneumatique qui aspire l'air de sa chambre.

9. Installation d'assemblage d'au moins deux capsules médicamenteuses (A, B ; A', B') par collage, **caractérisée en ce qu'**elle comprend :
- au moins un dispositif d'assemblage (1 ; 1') conforme à l'une quelconque des revendications précédentes,
- des premiers moyens (40) pour fournir une première des capsules à assembler (A ; A') à la matrice (10 ; 10') du dispositif alors que cette matrice est dans sa première configuration,
- des deuxièmes moyens (50) pour fournir une deuxième des capsules à assembler (B ; B') à la matrice du dispositif alors que cette matrice est dans sa première configuration,
- des moyens (60) d'application d'une colle (Z ; Z') sur la ou les capsules reçues par la matrice du dispositif alors que cette matrice est dans sa première configuration,
- des moyens (30) d'entraînement relatif entre, d'une part, le dispositif (1 ; 1') et, d'autre part, les premiers moyens (40), les deuxièmes moyens (50) et les moyens d'application (60), et
- des moyens de commande, notamment de commande pneumatique, pour passer la matrice (10 ; 10') entre ses première et seconde configurations.

10. Installation suivant la revendication 9, **caractérisée en ce que** les moyens d'entraînement relatif (30) sont adaptés, à la fois, pour placer la matrice (10; 10') accessible par le haut pour les capsules (A, B ; A', B') fournies par les premiers et deuxièmes moyens (40, 50), ainsi que pour la colle (Z ; Z') appliquée par les moyens d'application (60), et pour tourner la matrice vers le bas, d'une part, après que les moyens de commande aient passé cette matrice de sa première à sa seconde configuration et, d'autre part, avant que les moyens de commande passent cette matrice de sa seconde à sa première configuration.

## Patentansprüche

1. Vorrichtung (1, 1') zum Zusammenfügen von mindestens zwei Medikamentenkapseln (A, B; A', B') durch Kleben,
**dadurch gekennzeichnet, dass** sie aufweist:
mindestens eine Matrize (10, 10'), die zumindest teilweise gleichzeitig eine Aufnahme (15, 15') zum Ablegen der Kapseln (A, B, A', B') und eine Kammer (16, 16') begrenzt, wobei die Aufnahme und die Kammer durch eine verformbare Wand (14, 14') der Matrize voneinander getrennt sind, wobei die Matrize angepasst ist, um auf elastische Weise durch pneumatische Einwirkung ihrer Kammer (16, 16') aus einer ersten Konfiguration, in der ihre Aufnahme (15, 15') die Kapseln mit einem dazwischen angeordneten Klebstoff (Z, Z') aufnimmt, in eine zweite Konfiguration überzugehen, in der ihre verformbare Wand (14, 14') durch flexible Verformung die Kapseln zumindest teilweise umhüllt, um die Kapseln zumindest teilweise zwischen der verformbaren Wand und einem Boden der Aufnahme einzuspannen, um sie auszurichten und gegeneinander zu pressen.

2. Vorrichtung gemäß Anspruch 1, **dadurch gekennzeichnet, dass** die Matrize (10, 10') angepasst ist, um unter der Einwirkung eines pneumatischen Drucks, der die Kammer (16, 16') aufbläst, aus ihrer ersten in ihre zweite Konfiguration überzugehen.

3. Vorrichtung gemäß Anspruch 2, **dadurch gekennzeichnet, dass** die Aufnahme (15) einen konkaven Boden (15₁) zum Zentrieren der Kapseln (A, B) aufweist, der durch die Matrize (10) begrenzt ist, und dass, wenn sich die Matrize in ihrer ersten Konfiguration befindet, die verformbare Wand (14) auf der Seite der Aufnahme eine im Wesentlichen kegelstumpfförmige zu dem Boden der Aufnahme konvergierende Fläche (15₂) aufweist.

4. Vorrichtung gemäß einem der Ansprüche 2 oder 3, **dadurch gekennzeichnet, dass** sie einen steifen Einsatz (22') zum Aufnehmen und Verkeilen einer der Kapseln (A') aufweist, der in einem Sockel (12') der Matrize (10') eingerichtet ist, von dem aus sich die verformbare Wand (14) erstreckt, und der einen Boden für die Aufnahme (15') ausbildet.

5. Vorrichtung gemäß irgendeinem der Ansprüche 2 bis 4, **dadurch gekennzeichnet, dass** die verformbare Wand (14, 14') eine höhere flexible-Verformung-Kapazität als der Rest der Matrize (10, 10') aufweist, der zu diesem Zweck zum Beispiel von einer Aussteifung (28) umfasst ist und/oder eine größere Dicke hat und/oder durch den Einsatz (22') an Ort und Stelle gehalten wird.

6. Vorrichtung gemäß irgendeinem der Ansprüche 2 bis 5, **dadurch gekennzeichnet, dass** sie gleichzeitig aufweist: eine Stütze (20, 20') zum Befestigen der Matrize (10, 10'), die einen Durchgang (23, 23') zum Aufblasen der Kammer (16, 16') begrenzt, und eine pneumatische Verteilerplatte (25, 25'), an der die Befestigungsstütze (20, 20') montiert ist und die gemeinsam mehrere Aufblasdurchgänge (23, 23') versorgt, die in jeweilige Kammern (16, 16') münden ,die entweder durch ebenso viele einzelne Matrizen (10, 10') oder durch eine gemeinsame Matrize begrenzt sind.

7. Vorrichtung gemäß Anspruch 6, **dadurch gekennzeichnet, dass** die pneumatische Verteilerplatte (25') ferner mindestens einen Kanal (22'₂) zum Blasen der geklebten Kapseln (A', B') versorgt, der in jede Aufnahme (15') mündet.

8. Vorrichtung gemäß Anspruch 1, **dadurch gekennzeichnet, dass** die Matrize angepasst ist, um unter der Einwirkung eines pneumatischen Unterdrucks, der die Luft aus ihrer Kammer ansaugt, aus ihrer ersten Konfiguration in ihre zweite Konfiguration überzugehen.

9. Einrichtung zum Zusammenfügen von mindestens zwei Medikamentenkapseln (A, B, A', B') durch Kleben, **dadurch gekennzeichnet, dass** sie aufweist:
- mindestens eine Vorrichtung zum Zusammenfügen (1, 1') gemäß irgendeinem der vorhergehenden Ansprüche,
- erste Mittel (40) zum Bereitstellen einer ersten der zusammenzufügenden Kapseln (A, A') an die Matrize (10, 10') der Vorrichtung, während sich die Matrize in ihrer ersten Konfiguration befindet,
- zweite Mittel (50) zum Bereitstellen einer zweiten der zusammenzufügenden Kapseln (B, B') an die Matrize der Vorrichtung, während sich die Matrize in ihrer ersten Konfiguration befindet,
- Mittel (60) zum Aufbringen eines Klebstoffs (Z, Z') auf die von der Matrize der Vorrichtung aufgenommene Kapsel/aufgenommenen Kapseln, während sich die Matrize in ihrer ersten Konfiguration befindet,
- Mittel (30) zum relativen Antreiben zwischen einerseits der Vorrichtung (1, 1') und andererseits den ersten Mitteln (40), den zweiten Mitteln (50) und den Mitteln zum Aufbringen (60), und
- Mittel zum Steuern, insbesondere zum pneumatischen Steuern, um die Matrize (10, 10') zwischen ihrer ersten und ihrer zweiten Konfiguration zu bewegen.

10. Einrichtung gemäß Anspruch 9, **dadurch gekennzeichnet, dass** die Mittel zum relativen Antreiben (30) angepasst sind, um gleichzeitig die Matrize (10, 10') derart zu platzieren, dass sie für die Kapseln (A, B, A', B'), die von den ersten und den zweiten Mitteln (40, 50) bereitgestellt werden, sowie für den Klebstoff (Z, Z'), der von den Mitteln zum Aufbringen (60) aufgebracht wird, von oben zugänglich ist, und um die Matrize nach unten zu drehen, einerseits nachdem die Steuermittel die Matrize aus ihrer ersten in ihre zweite Konfiguration gebracht haben, und andererseits bevor die Steuermittel die Matrize aus ihrer zweiten in ihre erste Konfiguration bringen.

## Claims

1. A device (1; 1') for joining at least two medicine capsules (A, B; A', B') by adhesive bonding,
**characterized in that** the device comprises at least one matrix (10; 10') which at least partially delimits a recess (15; 15'), for depositing capsules (A, B; A', B') and a chamber (16; 16'), this recess and this chamber being separated from each other by a deformable wall (14; 14') of the matrix, which matrix is designed to move elastically, by pneumatic action of its chamber (16; 16'), from a first configuration, in which the recess (15; 15') of the matrix receives the capsules with a glue (Z; Z') interposed between them, to a second configuration, in which the deformable wall (14; 14') of the matrix at least partially envelops the capsules by flexible deformation, in such a way as to at least partially enclose these capsules between the deformable wall and a bottom of the recess in order to align them and press them against one another.

2. The device according to claim 1, **characterized in that** the matrix (10; 10') is designed to move from its first configuration to its second configuration by the action of pneumatic pressure that inflates the chamber (16; 16').

3. The device according to claim 2, **characterized in that** the recess (15) has a concave bottom (15₁) for centering the capsules (A, B), which is delimited by the matrix (10), and **in that**, when the matrix is in its first configuration, the deformable wall (14) has, on the side of the recess, a substantially tapered surface (15₂) converging toward the bottom of the recess.

4. The device according to one of claims 2 or 3, **characterized in that** the device comprises a rigid insert (22') for receiving and shimming one of the capsules (A'), which is arranged in a base (12') of the matrix (10'), from which the deformable wall (14) extends, and which forms a bottom for the recess (15').

5. The device according to any one of claims 2 to 4, **characterized in that** the deformable wall (14; 14') has a greater capacity for flexible deformation than the rest of the matrix (10; 10'), which, to that end, is for example cinched by a stiffener (28) and/or has a greater thickness and/or is kept in place by the insert (22').

6. The device according to any one of claims 2 to 5, **characterized in that** the device comprises both a support (20; 20') for fastening the matrix (10; 10'), which delimits a passage (23; 23') for inflating the chamber (16; 16'), and a pneumatic distribution plate (25; 25'), on which the fastening support (20; 20') is mounted and which jointly supplies several inflation passages (23; 23') emerging in respective chambers (16; 16') delimited either by an equal number of individual matrices (10; 10') or by a shared matrix.

7. The device according to claim 6, **characterized in that** the pneumatic distribution plate (25') also supplies at least one channel (22'₂) for blowing bonded capsules (A', B'), which emerges in each recess (15').

8. The device according to claim 1, **characterized in that** the matrix is designed to move from its first configuration to its second configuration by the action of a pneumatic vacuum that suctions the air from its chamber.

9. An installation for joining at least two medicine capsules (A, B; A', B') by adhesive bonding, **characterized in that** the installation comprises:
- at least one joining device (1; 1') according to any one of the preceding claims,
- first means (40) for providing a first of the capsules to be joined (A; A') to the matrix (10; 10') of the device while that matrix is in its first configuration;
- second means (50) for providing a second capsule to be joined (B; B') to the matrix of the device while that matrix is in its first configuration;
- application means (60) for applying a glue (Z; Z') on the capsule(s) received by the matrix of the device while that matrix is in its first configuration,
- driving means (30) for relative driving between the device (1; 1') on the one hand and the first means (40), the second means (50) and the application means (60) on the other hand, and
- control means, in particular pneumatic control means, for moving the matrix (10; 10') between its first and second configurations.

10. The installation according to claim 9, **characterized in that** the driving means (30) are designed both to place the matrix (10; 10') accessible from the top for the capsules (A, B; A', B') provided by the first and second means (40, 50), and for the glue (Z; Z') applied by the application means (60), and to turn the matrix downward, on the one hand, after the control means have moved that matrix from its first configuration to its second configuration and, on the other hand, before the control means move said matrix from its second configuration to its first configuration.
